# EUROPEAN PATENT SPECIFICATION

(11) **EP 1 440 144 B1**
(45) Date of publication and mention of the grant of the patent: **16.12.2009**
(21) Application number: 02792727.6
(22) Date of filing: 24.10.2002
(51) Int. Cl.: C12N 1/14, C12N 1/38, C12N 1/36, C12P 5/02, C12P 23/00, C12N 13/00, C12S 3/00

(54) **BLAKESLEA TRISPORA PRODUCING HIGH YIELD OF LYCOPENE IN A SUITABLE MEDIUM IN THE ABSENCE OF AN EXOGENOUS CAROTENOGENESIS INHIBITOR**
BLAKESLEA TRISPORA ZUR HERSTELLUNG VON LYCOPIN IN HOHER AUSBEUTE, IN EINEM GEEIGNETEN MEDIUM IN ABWESENHEIT EINES EXOGENEN COROTENOGENESE-HEMMSTOFFES
BLAKESLEA TRISPORA PRODUCTEUR A HAUT RENDEMENT DE LYCOPENE DANS UN MILIEU APPOPPRIE EN ABSENCE D'UN INHIBITEUR EXOGENE DE LA SYNTHESE DE COROTENOIDES

(30) Priority: 29.10.2001 RU 2001129106
(43) Date of publication of application: 28.07.2004
(73) Proprietor: DSM IP Assets B.V., 6411 TE Heerlen (NL)
(72) Inventor: SCHAAP, Albert, NL-2993 BG BARENDRECHT (NL); VAVILOVA, Ekaterina, Aleksandrovna, 113525 MOSCOW (RU); FLYAKH, Yadviga, Vitol 'dovna, 113525 MOSCOW (RU); MOROZOVA, Elena, Sergeevna, 121099 MOSCOW (RU); VINETSKI, Yuri, Pavlovich, 117463 MOSCOW (RU); IVLIEVA, Natalia, Alexandrovna, 129278 MOSCOW (RU); SEPEBRENNIKOV, Vladimir, Mikhailovich, 125057 MOSCOW (RU); VORONINA, Lilia, Nikolaevna, 111531 MOSCOW (RU); AKISHINA, Raisa, Illarionovna, 117526 MOSCOW (RU); GLASUNOV, Alexander, Viktorovich, 113208 MOSCOW (RU)
(74) Representative: Schwander, Kuno
(86) International application number: PCT/EP2002/012017
(87) International publication number: WO 2003/038064

(56) References cited:
- US-A- 3 369 974
- DATABASE WPI Section Ch, Week 199836 Derwent Publications Ltd., London, GB; Class D13, AN 1998-426078 XP002236404 & RU 2 102 416 C (URALBIOFARM STOCK CO), 20 January 1998 (1998-01-20) cited in the application
- MEHTA B. ET AL.: "Mutants of carotene production in Blakeslea trispora." APPLIED MICROBIOLOGY AND BIOTECHNOLOGY, vol. 42, no. 6, 1995, pages 836-838, XP009008257 cited in the application
- MEHTA B. ET AL.: "Lycopene cyclization in Blakeslea trispora." MYCOSCIENCE, vol. 40, 1999, pages 307-310, XP001146363 cited in the application
- DATABASE BIOSIS [Online] BIOSCIENCES INFORMATION SERVICE, PHILADELPHIA, PA, US1996 TERESHINA V M ET AL: "Effect of azines on lycopene formation in the mycelial fungus Blakeslea trispora." Database accession no. PREV199699237595 XP002236403 & PRIKLADNAYA BIOKHIMIYA I MIKROBIOLOGIYA, vol. 32, no. 4, 1996, pages 427-429, ISSN: 0555-1099

## Description

### Field of the invention

The invention relates to *Blakeslea trispora* (*B. trispora*) strain VKPM F-822 producing lycopene in a suitable medium in the absence of an exogenous carotenogenesis inhibitor, to a natural and efficient production process of lycopene by these strains, to a medium highly suited for this process and to an isolation process suited for any carotenoid compound, preferably for lycopene.

### Background of the invention

Lycopene is a red carotenoid. It is found at low levels in many organisms, being a biosynthetic precursor to most of the carotenoids found in Nature. However, sources that contain high levels of this natural dye are rare. Meanwhile, there is an increasing commercial interest in this compound because of its antioxidant and other potentially advantageous properties.

At present, several methods are known for the production of lycopene. The first method is a chemical synthesis (Meyer (2002), Chemie in unserer Zeit 36(3):178-192). The chemical method has the drawback of not yielding a product that is perceived as natural. A second method is the extraction from tomatoes (WO 97/48287 or EP 608027). Extraction from plants is a very expensive process due to the low concentration of lycopene in the tomato fruit.

Lycopene can also be produced via fermentation of *B. trispora. B. trispora* has been used for the production of β-carotene since the late 1950's. The first attempt at the fermentative production of lycopene is described in US 3,097,146. Therein, it is claimed that under specific favorable fermentation conditions, lycopene is produced by *B. trispora*, practically to the exclusion of other carotenoids. However, it is not clearly disclosed what these specific conditions would be, apart from a culture pH-value which is higher than 6.6 throughout the fermentation. The yield of lycopene is very low, with a
maximum level of 150 mg/l. No experimental data are shown to support the claim that the carotenoid produced is essentially pure lycopene.

Later approaches, have used specific chemical carotenogenesis inhibitors to prevent the formation of β-carotene and promote the accumulation of lycopene. Examples of this technology can be found in US 3,369,974, JP 48016189, JP 48016190, JP73016189, JP73016190, RU2102416, JP09313167, US3369974. By this method, lycopene concentrations up to 1030 mg/l have been achieved, with a concomitant 205 mg/l β-carotene being produced. Up to now, many compounds have been tested as inhibitors of lycopene metabolism, but no food-grade compounds have been found to be effective (Mehta & Cerdá-Olmedo (1999), Mycoscience 40:307-310).

It is known that there could be a problem regarding carotenoid production when the synthesis of β-carotene is blocked. Carotenogenesis is stimulated by trisporic acids, which are sexual hormones that are produced when the (+) and (-) strains are co-cultivated. At the same time, β-carotene are precursors of trisporic acids, and therefore blocking β-carotene production would amount to a decrease in the production of all carotenoids. WO 00/77234 teaches that the problem of stimulating carotenogenesis in the absence of sufficient β-carotene production may be solved by adding trisporic acids from an external source.This application describes a process for producing lycopene by fermentation of *B. trispora* producers of β-carotene or by mutants thereof that accumulate lycopene. Mutants that accumulate lycopene were already described in Mehta B et al., (1995, Appl. Microbiol. Biotechnol., 42:836-838). Such lycopene-producing mutant will be named hereafter the Mehta mutants. The Mehta mutants were obtained by mutagenesis and produced low yield of lycopene 200µg/g dry weight without adding any inhibitors on agar plates. Mehta concluded that the Mehta mutants are useless in biotechnological applications due to their poor lycopene yields. In WO00/77234, production of lycopene by this Mehta mutant was clearly inferior to the production obtained using the producers of β-carotene and having added imidazole as inhibitor, since the highest lycopene production by the Mehta mutant is about 4% of the production level that is obtained by using the producers of β-carotene and the inhibitor under the same conditions. Although volumetric productivities are not given, which makes comparison with prior art technology difficult, assuming a biomass production of 50 g/I (which is already a high estimate for fungal shake flask cultures), we calculate that a maximum concentration of about 60 mg/l of lycopene may have been produced by the Mehta mutant. This production estimation is still lower than what has been obtained earlier on in US 3,097,146 described above.

Hence, there is still a need for a more efficient method giving high yield of lycopene using a natural fermentation and isolation process. The present application provides such a method: production of lycopene by a novel strain of *B*. *trispora* in a microbiological suitable medium, which does not contain any exogenous specific carotenogenesis inhibitors, that only contains ingredients commonly employed in fermentation. The advantages of the strain of the invention is that it allows to produce lycopene in a fermentation process, wherein no compounds are present that would compromise the natural character of the process, no products are added that would compromise the food-grade character of the lycopene produced.

### Detailed description of the invention

In contrast with the methods known in the art for production of lycopene, the present application provides for a high production of lycopene on a suitable medium commonly used in fermentation, without any external additions to inhibit the carotenogenic pathway. This is achieved by the use of a mutant, obtained by chemical mutagenesis and selection. In view of the characteristics of mutants previously found by classical mutagenesis (1995, Appl. Microbiol. Biotechnol., 42:836-838), it is highly surprising that such a mutant can be found.

The invention first relates to strain VKPM F-822 of *Blakeslea trispora*, which is able to produce at least 0.3 g/l lycopene, when co-cultivated for at least three days with a suitable strain of *Blakeslea trispora* of the opposite mating type in a suitable medium in the absence of an exogenous specific carotenogenesis inhibitor. Preferably at least 0.5 g/I of lycopene is produced, preferably at least 0.7, preferably at least 0.8, more preferably at least 1.0, most preferably at least 1.2 and even most preferably at least 1.5 g/I. The amount of lycopene produced is measured at the end of the fermentation process.

A suitable medium means a medium, which only comprises ingredients commonly used in fermentation media. Thus, no compounds are present that would compromise the natural character of the process and/or no products are added that would compromise the food-grade character of the lycopene produced and/or no expensive additives are added.

This medium does not comprise any exogenous carotenogenesis inhibitors. In the context of the invention, a carotenogenesis inhibitor is a substance, which specifically prevents the formation of β-carotene and promotes the accumulation of lycopene. A carotenogenesis inhibitor can also be defined as a substance specifically inhibiting the metabolism of lycopene. The only specific carotenogenesis inhibitors that may be present during the fermentation are those possibly produced by the strain themselves. Examples of carotenogenesis inhibitors are those described in WO200077234, US 3,369,974, JP 48016189 and JP 48016190, Mehta & Cerdá-Olmedo (1999), Cerdá-Olmedo & Hütterman (1986) Angewandte Botanik 60:59-70, JP73016189, JP73016190, RU2102416, JP09313167, US3369974.These inhibitors may be N-heterocyclic compounds, such as imidazole, pyridine, morpholine and their substituted derivatives, or tertiary-amino compounds. In the context of this application, thiamin would not be considered as a tertiary amine. Thiamin is an essential vitamin for *B. trispora*, and therefore should be present in the fermentation medium.

Preferably, a carotenogenesis inhibitor will be able to prevent at least 10% w/w of the formation of β-carotene and concomitantly promote at least 10% w/w of lycopene formation as compared to the situation, wherein no carotenogenesis inhibitor is present. Preferably, at least 20% of β-carotene formation is prevented, more preferably at least 30%, even more preferably at least 40%, most preferably at least 50% and even most preferably at least 60%, at least 70%, at least 80%, at least 90%, at least 95%, at least 99%.

Preferably, trisporic acids are not added during fermentation. If trisporic acids are not added during fermentation, the only source of trisporic acids, which may be present are the native trisporic acids produced by the strains themselves.

Preferably, a suitable medium comprises one or more sources of carbon, one or more sources of nitrogen, mineral salts and thiamine. The sources of carbon that can be added are simple or complex nutrients and include carbohydrates or fats, such as dextrins, starches, glucose, saccharose, fructose, maltose, maltodextrins, animal or vegetable oils. The suitable medium preferably has an assimilable nitrogen source, organic or inorganic, such as soya flour, maize flour, soluble distillates, yeast extract, cottonseed flour, peptone, casein, ammonia, ammonium salts, nitrate, corn extract, corn steep solids and corn steep liquor, etc. The mineral salts that are preferably present in the suitable medium include phosphates, sulphates, chlorides, sodium, potassium, ammonium, calcium and magnesium. The proportion of the nutrients may be determined on the basis of the growth requirements of the *B*. *trispora* strain and on the lycopene production levels. Fermentation is preferably carried out in aerobic conditions and in submerged culture. Preferably, the suitable medium comprises a high content of sugar as C-source as defined further in the application. The most preferred medium is defined below in Table 1. The fermentation process may be carried out at a temperature comprised between 20 and 34 °C. Alternatively, the fermentation process may be carried out at two or more different temperatures within this range, to allow independent optimization of subsequent phases of the process. Preferably, the temperature may be in a first step of the fermentation chosen to be suited for optimal growth of the host, and in a second step suited for optimal production of lycopene by the host. More preferably, the temperature suited for production of lycopene is lower than the temperature suited for growth of the host. Both temperatures are between 20 and 34 °C. Preferably the temperature suited for growth is between 25 and 32 °C, more preferably is 32 °C and the temperature suited for lycopene production is between 20 and 25 °C, more preferably is 22°C.

Alternatively, the fermentation process is a fed-batch fermentation process. In such a fed-batch fermentation process, one or more nutrients are supplied by feeding the fermentation system during the fermentation, in addition to a supply of those nutrients in the suitable medium at the start of the fermentation. Alternatively, one or more nutrients are supplied by feeding the fermentation system during fermentation; thereby replenishing nutrients consumed during fermentation. One of the advantages of a fed-batch system is that undesirable high concentrations of nutrients such as sugar can be avoided. Another example of a potentially harmful nutrient that can be supplied by a feed is ammonia, or any other nutrient for which high concentrations are undesirable. A second advantage of fed-batch fermentation is the possibility of prolongation of the fermentation after the moment that one of the nutrients has become exhausted, by feeding such a nutrient. The use of a fed batch process often results in higher biomass concentration and higher product concentration.

The concentration of the carotenoids present at the end of the fermentation process can be measured spectrophotometrically after extraction of the carotenoids, taking into account the characteristic absorption maxima of the various carotenoids. Alternatively, the carotenoids may be separated by HPLC or other chromatographic methods, after which detection may occur at a common absorption wavelength, or again at different wavelengths specific for the various carotenoids. Specific attention may be paid to the occurrence of stereoisomers of carotenoids, which may have different chromatographic or spectral properties. It is known in the art that the primary product of the biosynthetic pathways is the trans-isomer, and that isomerisation to different cis-isomers may occur to various extents during sample processing or product isolation. Hence, the total production level of a carotenoid species is considered to be the sum of the levels of all its stereoisomers.

It may be difficult to detect all isomers of all carotenoids. Hence, it may be more convenient to characterize carotenogenic mutants by the ratio of two well-detected products. In the context of the present application, the two most important carotenoids are β-carotene, being the main product of the parent strain, and lycopene, being the main product of the mutants. Mutants or process conditions may be characterized by the ratio of lycopene and β-carotene present in relevant samples. Alternatively, the mutants may be characterized by the amount of total lycopene produced as percentage of total carotenoids present. As a consequence, in the context of the application (unless otherwise indicated) a concentration of lycopene, respectively β-carotene corresponds to the sum of the concentration of cis and trans lycopene and cis and trans β-carotene respectively. Due to the presence of multiple carotenoids, the sum of β-carotene and lycopene is approximatively 90% w/w of the total amount of carotenoids produced by the strain (unless indicated otherwise).

The strain used is able to produce at least 70% w/w lycopene and less than 20% w/w β-carotene, when co-cultivated for at least three days with a suitable strain of *Blakeslea trispora* of the opposite mating type in a suitable medium in the absence of an exogenous specific carotenogenesis inhibitors, preferably at least 75% and less than 15%, more preferably at least 80% and less than 10%, most preferably at least 85% and less than 5%. The percentages given here represent the percentage of lycopene respectively β-carotene as percentage of the total amount of carotenoids produced by the strain.

The strain used produces at least 70% w/w lycopene, when co-cultivated for at least three days with a suitable strain of *Blakeslea trispora* of the opposite mating type in a suitable medium in the absence of an exogenous specific carotenogenesis inhibitors, preferably at least 75%, more preferably at least 80%, most preferably at least 85%. The percentages given here represent the percentage of lycopene as percentage of the total amount of carotenoids produced by the strain.

The strain used produced a carotenoid mixture, in which the ratio of lycopene to β-carotene is higher than 4:1, when this strain has been co-cultivated for at least three days with a suitable strain of *Blakeslea trispora* of the opposite mating type in a suitable medium in the absence of an exogenous specific carotenogenesis inhibitors. In yet another preferred embodiment, the strain produced carotenoid mixtures with lycopene as principal component. In yet another preferred embodiment, the strain produced mixtures of carotenoids that comprise the biosynthetic pathway from phytoene to β-carotene.

Carotenoid mixtures are provided, in which the ratio of lycopene to β-carotene is higher than 4:1. Preferably, carotenoid mixtures with lycopene as principal component are provided. In yet another preferred embodiment, mixtures of carotenoids that comprise the biosynthetic pathway from phytoene to β-carotene are provided. In yet another preferred embodiment, a biomass containing a mixture of carotenoids as defined above is provided. Accordingly, this biomass can further be used in feed, food or cosmetic applications.

The most preferred carotenoid mixtures provided comprise:
0-5 w % Neurospoene, 0-5 w % Phytoene, 0-10 w % β-carotene, 5-20 w % β-carotene, 70-90 w % lycopene. Even most preferred mixtures comprise 0-2 w % Neurosporene, 0-2 w % Phytoene, 0-5 w % β-carotene, 5-15 w % β-carotene, 80-90 w % lycopene.

A strain may be obtained by chemical mutagenesis and selection. Various methods of mutagenesis are known in the art (see for instance Rowlands (1984), Enzyme Microb. Technol. 6:3-10). One of these methods is the treatment with mutagenic chemicals, including nitroso-guanidine (NTG), ethyl-methane-sulfonate (EMS), or nitroquinoline oxide (NQO). Another method is treament with mutagenic radiation, such as UV-light, gamma-rays or X-rays. With respect to the selection method, selection for deviating colony color upon cultivation on agar media is particularly powerful for carotenogenic mutants, but other criteria may be used as well such as colonial and mycelial morphology, sporulation characteristics, growth rate, resistance against inhibitory compounds or stress conditions, and other methods commonly used in strain improvement programmes.

The lycopene-producing strain is *Blakeslea trispora lye26*(-). This strain was obtained through multiple rounds of mutagenesis and selection, using 1-methyl-3-nitro-nitrosoguanidine (NG) and gamma-rays as mutagenic factors, starting from the β-carotene-producing parent strain *B*. *trispora 111* (-). When grown in co-culture with a suitable (+) strain of the species, such as a strain used in β-carotene production, in a suitable growth medium as defined above, strain *lyc26*(-) will produce lycopene as the most abundant carotenoid, accounting for more than 70% of total carotenoids. *Blakeslea trispora lyc26*(-) has been deposited at the All-Russian Collection of Commercial Microorganisms as *Blakeslea trispora* VKPM F-822, and is thus available to the public. Lycopene is the primary carotenoid produced on all growth media tested by strain *lyc26*(-). Preferably the mutants of the application are cultivated on a medium having a high content of sugar as C- source as defined further on in the application. Most preferably, the mutants are cultivated on the medium presented in Table 1. Preferably, the strain used has essentially the same characteristics as the strain deposited under VKPM F-822.

The strain used is derivable from the strain deposited under VKPM F-822 or is a progeny thereof.

The application further relates to the use of the strain as defined above for producing lycopene, preferably by using the process defined in example 8.

According to a further aspect of the application, there is provided a suitable medium having a high content of sugar as C-source. A high content of sugar as C-source means a high content of readily assimilable sugars as C-source. Such a medium is highly suited for the production of lycopene by *B. trispora* mutants. In this respect, readily assimilable sugars are defined as being mixtures consisting mainly of monosacharides and disaccharides. Examples of such C-sources are dextrose, glucose, fructose or high-maltose syrups. In contrast, production media traditionally employed for β-carotene production comprise C-sources that contain starch as the main assimilable sugar source, such as soy bean flour or corn flour, for example the medium described in Table 3. Alternatively, media have been used that do contain readily assimilable sugar sources, but in combination with other C-sources, such as plant oils, for example the medium described in Table 11. The rationale behind these media designs is that readily assimilable carbon is believed to repress the formation of carotenoids, although it is very suited for growth. Surprisingly, it was found that lycopene production by mutants of *B*. *trispora* was highly efficient in production media containing readily assimilable sugars as their main carbon source. Preferably, these media contain at least 50% of their carbon by weight in the form of readily assimilable sugars, more preferably at least 60%, even more preferably at least 70%, and most preferably at least 80%. In these percentages, the carbon contained in the nitrogen source has also be taken into account. Preferably, these media contain at least 5% w readily assimilable sugars, more preferably at least 6% w and most preferably at least 10% w.

Most preferably, the medium is as defined below in Table 1.

The pH throughout the fermentation is not found as being a critical parameter influencing lycopene production. The pre-sterilization pH may be ranged between 6.2 and and 8.4, preferably between 7.5 and 8.4.

**Table 1: Optimized medium (all ingredients in g /100 ml):**

| | |
|---|---|
| maltose syrup | 8.0-14.0 |
| corn extract | 4.0-8.0 |
| baker's yeast (dry) | 0.03-0.07 |
| KH₂PO₄ | 0.04-0.06 |
| NaCl | 0.1-0.5 |
| MnSO₄.7H₂O | 0.005-0.015 |
| thiamin | 0.0002 |
| vegetable oil | 3.0-6.0 |
| tap water | 84.8-71.4 |
| pre-sterilization pH | 6.0-8.4 |

The application is also related to the use of this preferred medium as defined above or preferably as defined in Table 1 to produce lycopene using a suited host.

Another aspect of the application relates to a process for producing lycopene using the strain as defined above, co-cultivating it for at least three days with a suitable strain of *Blakeslea trispora* of the opposite mating type in a suitable medium. Preferably, the suitable medium used is the suitable medium as defined earlier in the description. The process is performed in the presence of an exogenous specific inhibitor of carotenogenesis such as those described above. According to another preferred embodiment, the process is performed in the presence of exogenous trisporic acids. More preferably, the process is performed using the optimized medium defined above in Table 1.The cultivation may be performed in multiple stages, for instance separate vegetative and production stages. Vegetative stage may be separate for each mating type, followed by a joint production culture. Also, the production culture may be started with only one of the mating type, the other one being added later at the start of the actual production phase. Preferably, the strains are co-cultivated for at least two days, more preferably at least three days, even more preferably at least four days and most preferably at least five days.

After the co-culfivation, the broth may be inactivated, for instance by pasteurization, and the biomass is collected, for instance by filtration. Subsequently, the biomass can be further stabilized by drying, vacuum drying, fluidized bed drying or belt drying.

The lycopene is not separated from the mycelium. The biomass containing a mixture of carotenoids as defined above is provided. Accordingly, this biomass can further be used in feed, food or cosmetic applications.

Alternatively and more preferably, the lycopene is separated from the wet or dry mycelium by any method that has been described for cell rupture that makes it possible to release the cell content. The lycopene may be further dissolved in any solvent in which it is soluble. Two ways of separating lycopene from the mycelium are preferred: extraction, or isolation. Preferably, this can be done by extraction with a suitable solvent, such as alcohols, ketone or esters, such as acetone or ethyl-acetate, alkane such as n-hexane. Alternatively, this may be done by supercritical extraction, for instance with CO₂, possibly with an organic solvent functioning as co-solvent.

Alternatively and more preferably, this is done by a direct isolation method, characterized by the use of a water-immiscible solvent, and isolation of the carotenoid-containing interface layer as described in WO 01/55100. Preferably, the direct isolation method used is the one defined hereafter.

According to a further aspect of the application, there is provided a direct isolation process, which is a process for the preparation of a crystalline carotenoid compound, said process comprises the following steps:
(a) disruption of carotenoid-containing cells, preferably from a microbial source,
(b) washing the crystalline carotenoid disrupted cell mixture with a solvent suitable to remove lipid, preferably ethyl acetate, more preferably n-butanol, and separation of the carotenoid containing interface,
(c) treatment of the appropriate crystalline carotenoid layer obtained at step (b) with alkali at a pH of 9-12 and at a temperature of 10-95°C, preferably 30-85°C, most preferably 50-75°C, optionally in the presence of a lower alcohol,
(d) optional addition of a salt to the alkali-treated crystalline carotenoid suspension,
(e) optional separation of the crystalline carotenoid suspension from the liquid phase,
(f) optionally washing the crystalline carotenoid suspension with a salt containing aqueous solution,
(g) optionally washing the crystalline carotenoid suspension with an aqueous acidic solution; the pH of the washing solution preferably has a value between 1 and 5, more preferably between 2 and 4, optionally in the presence of a lower alcohol. The ratio of lower alcohol to water in the lower alcohol / water mixture preferably is between 5:1 and 1:5, more preferably between 1:1 and 1:2.
(h) washing the crystalline carotenoid suspension in a first washing procedure with a lower alcohol, wherein the order of performance of steps (b)-(e) and (f) is arbitrary,
(i) washing the crude carotenoid crystals resulting from the (a)-(f) process steps in a second washing procedure with water or with a mixture of a lower alcohol and water,
(j) washing the crystals with a fresh solvent, and
(k) drying the crystals.

Carotenoid-containing microbial cells may be obtained from any suitable fermentation process of a carotenoid-producing microorganism of choice. The carotenoid-containing microorganism may be a bacterium, a fungus, an alga or a yeast. Preferably, it is a fungus of the order *Mucorales*, preferably *Blakeslea trispora*, an alga of the genus *Dunaliella*, or a yeast of the genus *Phaffia*, preferably *Phaffia rhodozyma.* More preferably, the *Blakeslea trispora* mutant defined earlier is the carotenoid-containing microorganism. This process allows the isolation of any known carotenoid compounds. Preferred carotenoid compounds are lycopene, β-carotene, phytoene and astaxanthin.

### Step (a)

The resulting fermentation broth comprising microbial cells and fermentation fluid may be used directly for the isolation of carotenoid crystals. Alternatively, prior to carrying out the process of the application, microbial cells may be separated from the fermentation fluid by any suitable method, such as filtration or centrifugation.

The carotenoid-containing microbial cells are opened by disrupting the cell walls by means of mechanical, chemical and/or enzymatic treatment. For instance, the cells may be subjected to homogenization, sonication, autolysis, osmolysis and/or plasmolysis, optionally with addition of suitable agents such as detergents, acids, bases, enzymes, autolysis-enhancing substances, osmolysing agents such as salts, and/or plasmolysing agents. In this way, a crystalline carotenoid disrupted cell mixture is released from the cells.

### Step (b)

The crystalline carotenoid disrupted cell mixture thus obtained is then further purified.

To improve the yield of the recovery process, the crystalline carotenoid disrupted cell mixture is washed with a solvent suitable to remove a.o. lipids, preferably an organic solvent, not miscible with water in which the crystalline carotenoid has a low solubility. This solvent is added in an amount of 1% to 100% of the amount of crystalline carotenoid suspension, preferably 10 % to 40%, more preferably 20% to 30%. It should be noted that the amount of solvent necessary for lipid removal is substantially lower than the amount of solvent necessary for solvent-extraction of a carotenoid from microbial biomass. A preferred solvent not miscible with water is hexane, or ethyl acetate. Preferably the solvent is ethyl acetate. Another preferred solvent is an alcohol such as isopropanol or n-butanol. N-butanol is also denominated as 1-butanol. Most preferably, the solvent is n-butanol.

Washing, as referred to in this application, includes a step of suspending or stirring the material to be washed in a suitable amount of the solvent of choice and a step of decantation or centrifugation and subsequent recovery of the appropriate layer.

### Step (c)

The next purification step consists of the treatment of the appropriate crystalline carotenoid layer obtained at the former step with alkali. The alkali treatment comprises the addition of an alkaline aqueous solution having a pH between 9 and 12 to the carotenoid layer and the subsequent incubation, preferably under stirring, for an appropriate time period at a temperature between 10-95°C, preferably between 30-85°C, more preferably between 50-75°C. The ratio of alkaline solution to the carotenoid layer conveniently may vary from about 5:1 to about 1:1 (w/w). The duration of the alkali treatment typically will depend on the applied pH and temperature, in the sense that the lower the pH and temperature applied during treatment, the longer the treatment should be. For instance, the alkali treatment may be performed during 2 hours at pH 12 and a temperature of 75°C or during 8 hours at pH 10 and a temperature of 50 °C. Optionally, the alkali treatment may be carried out in the presence of a lower alcohol.

### Steps (d), (e), (f)

After alkali treatment, a water-soluble salt, such as sodium chloride, more preferably sodium acetate, may optionally be added to the alkali-treated crystalline carotenoid layer. The crystalline carotenoid layer may then be separated from the liquid phase and may optionally be washed with a salt containing aqueous solution. The separation of the crystalline carotenoid layer may be carried out by any method known in the art, such as filtration, centrifugation or cooling.

Before applying the next purification step, the crystalline carotenoid layer may be washed one or more times with water.

### Step (g)

Optionally, the crude carotenoid crystals may further be purified by applying a second washing procedure comprising washing the crystals one or more times with water or with a mixture of a lower alcohol and water. The pH of the washing solution preferably has a value between 1 and 5, more preferably between 2 and 4. The water may be acidified with any suitable acid, such as sulphuric acid or hydrochloric acid or with an acidic buffer solution, such as a borate/hydrochloric acid or a citrate/ hydrochloric acid buffer. The ratio of lower alcohol to water in the lower alcohol / water mixture preferably is between 5:1 and 1:5, more preferably between 1:1 and 1:2.

The crystals are subsequently separated from the liquid phase by any suitable method, such as filtration or centrifugation.

### Steps (h), (i)

The crystalline carotenoid layer is then subjected to a first washing procedure comprising washing the suspension with a lower alcohol, yielding crude carotenoid crystals. This first washing procedure may be repeated one or more times.

Typically, a washing step performed according to the application includes strirring the crystalline carotenoid layer or the (crude) carotenoid crystals with the washing liquid and subsequently separating the crystalline carotenoid layer or the crystals from the liquid phase.

Preferably, the process steps (a)-(g) as described above are performed in the order (a), (b), (c), (d), (e), (f), (g). Preferably the process steps (a)-(g) as described above are performed in the order (a), (g), (b), (c), (d), (e), (f).

Throughout the present application, a lower alcohol is defined as a (C₁₋₆) alcohol, for instance methanol, ethanol, 1-propanol, 2-propanol and 1-butanol. The lower alcohol as used in the present application may be a single alcohol or may be a mixture of two or more alcohols. Preferably, the lower alcohol is ethanol, 1-butanol or a mixture of 1-butanol and ethanol.

### Step (j)

Hereafter, the crystals are washed one or more times with a fresh solvent. The fresh solvent is a lower alcohol, preferably ethanol, or an acetate ester of a lower alcohol, preferably ethyl acetate. More preferably, the fresh solvent is a food-grade solvent.

Preferably, the lower alcohol used in the first two washing procedures of the crystalline carotenoid suspension and the fresh solvent used in the final washing step of the crystals are the same solvent.

### Step (k)

In a final step of the process of the application, the crystals are dried.

An important advantage of the present application as compared to conventional extraction/crystallisation processes for the isolation of a crystalline carotenoid is that the use of organic solvent for the extraction of the carotenoid is avoided. As a consequence, substantially no solvent is incorporated in the crystal lattice of the resulting carotenoid compound.

In another aspect of the application, the process of the present application is used to increase the carotenoid content of any crude crystalline carotenoid composition, preferably of any crude crystalline carotenoid suspension.

The invention is further illustrated by the following examples.

### Examples

### Example 1: Strain isolation and characteristics

Strain *Blakeslea trispora lye26*(-) was obtained through multiple rounds of mutagenesis and selection, using 1-methyl-3-nitro-nitrosoguanidine (NG) or gamma rays as mutagenic factors, starting from the β-carotene-producing parent strain *B*. *trispora 111* (-). For mutagenic treatment, spore suspensions were prepared (10⁶-10⁷ spores per ml) in a solution of 10% glycerol and 5% lactose in water. For NG mutagenesis, a solution of 150 µg/ml NG in a 100 mM Tris-HCl buffer (pH= 8.0) was used. For gamma-ray mutagenesis, a dose of about 1 Mrad was used. In both methods, mutagenic exposure was aimed at achieving 1 % survival of spores. Lycopene-producing mutants were selected by their red color characteristic, when cultivated on wort agar plates.

Strain *lyc26*(-) exhibits the following morphological features:
On wort agar:
   colony growth: restrained
   aerial mycelium: slightly downy, interlaced, light crimson colored
   spore formation: poor
On SM 17-1 agar:
   colony growth: restrained with planar colonies
   aerial mycelium: dense, pink colored, slightly raised center
   spore formation: very poor

The features of the *lyc26* (-) strain are stable. The strain is nonpathogenic.

The SM 17-1 agar has the following composition as presented in Table 2.

**Table 2: SM 17-1 medium (concentrations in g/l)**

| | |
|---|---|
| Glucose | 3 |
| MgSO₄.7H₂O | 0.05 |
| L-asparagine | 0.2 |
| KH₂PO₄ | 0.2 |
| yeast extract | 0.1 |
| Na-deoxycholate | 0.1 |
| thiamine | 0.00002 |
| agar (Difco) | 20 |

### Example 2: Lycopene production on agar plates

On modified SM 17-1 agar, using a wide range of mono- and disaccharides (e.g. glucose, arabinose, fructose, lactose, maltose) as a source of carbon, the mycelium of *B. trispora* strain strain *lyc26* (-) was crimson-colored when the colonies were overlayed with filter disks impregnated with an extract of a β-carotene production culture. Apparently, the trisporic acids produced in the mated culture of *B. trispora* (+) and (-) strains used for the β-carotene production, were effective in stimulating lycopene production in the mutant *lyc26* (-). The strains used for the β-carotene production culture were the parent strain 111 (-) and the (+) strains VKPM F-820 or VKPM F-821.

### Example 3: Lycopene production in shake flasks

A corn-soya medium, known in the art as suitable for β-carotene production, was used for lycopene production with *B*. *trispora* strain *lyc26* (-), in joint culture with a suitable *B*. *trispora* (+) strain. In this example, strain VKPM F-820 and VKPM F-821 were used as (+) strains.

The (-) and (+) strains were cultured separately on wort agar slants at a temperature of 28 °C for 20 hours in the dark, and subsequently for 8-12 days under illumination with daylight lamps at a temperature of 22 °C. To obtain the seeding material, aerial mycelium and spores were washed off the agar surface with distilled water.

The suspensions so obtained were used to seed 750 ml Erlenmeyer flasks containing 50 ml of a liquid medium composed as given in Table 3.

**Table 3: Liquid medium (all ingredients as % w/v)**

| | |
|---|---|
| corn flour | 4.7 |
| soy bean flour | 2.3 |
| KH₂PO₄ | 0.05 |
| thiamine | 0.0002 |
| tap water | balance |
| pH | Adjusted to 6.2 - 6.7 before sterilization with NaOH |
| sterilization | 45 minutes at 120 °C |

The (-) and (+) strains were cultured separately on a rotary shaker (250 rpm) for 48 hours at a temperature of 26-27 °C. The culture suspensions thus obtained were used to inoculate 750 ml production flasks, containing 50 ml of a production medium (1) with the following composition as given in Table 4

**Table 4: Production medium (1) (in % w/v).**

| | |
|---|---|
| corn flour | 1.73 |
| soy bean flour | 4.0 |
| KH₂PO₄ | 0.05 |
| thiamine | 0.0002 |
| sunflower oil | 8.0 |
| tap water | balance |
| pH | adjusted to 6.1 - 6.5 before sterilization with NaOH |
| sterilization | 45 minutes at 120 °C |

For inoculation, 10 ml of (-) strain culture and 1 ml of (+) strain culture was used. Subsequently, the production flasks were incubated on a rotary shaker (250 rpm) for 5 days at 26-27 °C.

After 5 days, a 0.5 ml sample of culture broth was diluted with 1 ml water, and centrifuged. The pellet was resuspended in 1 ml of 96% ethanol, and centrifuged. The pellet was collected and homogenized in a Potter tube with chloroform. The chloroform was collected and the homogenization was continued with fresh chloroform. This was repeated until color was no longer taken up by the chloroform fraction. The collected chloroform fractions were pooled, and the total volume was adjusted to 10 ml with chloroform. The production levels of β-carotene and lycopene were determined by HPLC (Table 5). Samples for the analysis were prepared by diluting 0.5 ml of the chloroform extract with 95 ml acetone. In this Example, the presence of β-carotene and lycopene was measured. The presence of other minor carotenoids was not measured as we did in Example 6. The lycopene content as calculated in last column of Table 5 represents the ratio between the amount of lycopene produced to the amount of lycopene and β-carotene produced.

**Table 5: Influence of the mating strain on the production level of β-carotene and lycopene in production medium (1)**

| *Blakeslea trispora* strains | | Concentration (g/l) β-carotene lycopene | | Lycopene content, % of the aggregate carotenoids synthesized |
|---|---|---|---|---|
| (-) | (+) | | | |
| *lyc26* | VKPM F-820 | 0.05 | 0.31 | 86.0 |
| *lyc26* | VKPM F-821 | 0.18 | 0.74 | 80.0 |

It is clear that the mutant *lyc26* (-) produces lycopene as main carotenoid product, its lycopene production being 4-5 times higher than its β-carotene production, irrespective of the (+) strain used for the mated culture. It is also clear that the absolute level of the carotenoids produced by the mutant depends much more on its interaction with a suitable (+) strain, and that different (+) strains may give more or less favorable results.

In a comparative experiment, the lycopene and β-carotene production of the β-carotene-producing parent strain 111 was tested, using the same set of (+) strains and the same experimental conditions. The lycopene production of strain 111 was less than 0.03 g/I. The β-carotene production of strain 111 was between 2.8 and 3.4 g/I.

### Example 4: Lycopene production in improved medium

The preparation of the separate seed cultures for the (+) and (-) strains was performed as in Example 3, using the same strains. However, this time the precultures of the (+) strains were incubated for 24 hours only. These suspensions of the (-) and (+) strains were used to inoculate a production medium (2) with the following composition as presented in Table 6. :

**Table 6: Production medium (2) (in % w/v)**

| | |
|---|---|
| maltose syrup | 12.0 |
| corn extract | 6.0 |
| Baker's yeast (dry) | 0.05 |
| KH₂PO₄ | 0.05 |
| NaCl | 0.3 |
| MnSO₄ 7H₂O | 0.01 |
| thiamine | 0.0002 |
| vegetable oil | 4.0 |
| tap water | balance |
| pre-sterilization pH | 8.0 |
| sterilization at 110°C for 30 min. | |

The incubation conditions of the production cultures and the analytical procedures were as in Example 3. The results are given in Table 7. In this Example, the presence of β-carotene and lycopene was measured. The presence of other minor carotenoids was not measured as we did in Example 6. The lycopene content as calculated in last column of Table 7 represents the ratio between the amount of lycopene produced to the amount of lycopene and β-carotene produced.

**Table 7 : Influence of the mating strain on the production of β-carotene and lycopene in production medium (2)**

| *Blakeslea trispora* strains | | Concentration (g/l) β-carotene lycopene | | Lycopene content, % f the aggregate carotenoids synthesized |
|---|---|---|---|---|
| (-) | (+) | | | |
| *lyc26* | VKPM F-820 | 0.14 | 1.74 | 93.0 |
| *lyc26* | VKPM F-821 | 0.03 | 1.41 | 98.0 |

It is clear that production medium (2) supports much higher lycopene production levels by the mutant than production medium (1) (compare Table 5 and 7). Production medium (2) is also better than production medium (1) as to the ratio of lycopene to β-carotene (compare Table 5 and 7). In this example, the (+) strain VKPM F-820 used for the mating gives much higher lycopene production than the VKPM F-821. In Example 3, the VKPM F-821 (+) strain produced the most favorable results as to the concentration of lycopene produced. This may well be due to the known problem of optimizing the ratio of (+) to (-) strains: different strain may have different growth rates in different media, which may be expected to lead to differences in the actual amount of biomass of each strain present in the production phase and therefore to differences in terms of lycopene yield. It is within the ordinary competence of those skilled in the art to optimize the ratio of the mated strains once the other experimental conditions, such as the medium have been chosen.

In a comparative experiment, the lycopene production of the β-carotene-producing parent strain 111 was tested in this improved medium, using the same set of (+) strains and the same experimental conditions. The lycopene production of strain 111 was not more than 20-30 mg/l, whereas β-carotene-production levels by the parent strain reached 2.5 - 3.2 g/I in this medium.

We conclude that the production of β-carotene by the parental strain 111 is not influenced by the medium used (compare with example 3). This improved medium is highly suited for the production of lycopene by the *lyc 26* mutant.

### Example 5. Lycopene production in a 10L fermenter.

The preparation of the separate seed cultures for the (+) and (-) strains was performed as in Example 3, except that 2000 ml flasks were used, filled with 200 ml of the medium as defined below. Multiple flasks were used to obtain sufficient inoculation material for the fermenter cultures.

The production medium (3) was based on that used in Example 4, but with some modifications, using industrially available raw materials to allow easier scaling up to larger volumes as given in Table 8.

**Table 8: Production medium (3) (concentrations in g/kg)**

| | |
|---|---|
| Sweet M syrup (Cerestar) | 75 |
| 80% dry matter (68% of which is maltose) | |
| corn steep solids | 30 |
| Engevita dry inactive yeast (DSM) | 5 |
| KH₂PO₄ | - |
| NaCl | 3 |
| MnSO₄ 7H₂O | 0.1 |
| thiamine | 0.002 |
| soy oil | 5 ml |
| tap water | balance |
| pre-sterilization pH | adjusted to pH= 6.7 with NaOH |
| sterilization at 120°C for 120 min. | |

The production culture was performed in a 10L-liter fermenter with an initial medium content of 4 kg at a temperature of 32 °C, an agitation speed of 200 rpm, and an aeration of 6 L per minute. An antifoaming agent Basildon (Basildon Chemical Co., Abingdon, UK) was added when needed and the dissolved oxygen was always above 25% saturation. The medium was inoculated with a flask containing a mixture of 1000 g of mature seed culture of the (-) strain, 500 g of mature seed culture of the (+) strain and 120 g sterile soy oil. After inoculation, the culture's temperature was kept at 32 °C for 48 hours. After adjustment of the temperature to 22 °C, the culture was prolonged for another 72 to 120 hours. The pH of the culture was not controlled. The starting value was 6.2, and subsequently the pH rose until it reached 6.8 at the end of the fermentation.

Samples were drawn at regular time intervals for analysis. A part of the sample was used directly for spectrophotometric analysis of the lycopene and for the dry matter content of the broth. The other part of the sample was homogenized, and frozen at -20 °C to be used later in a more detailed analysis of the carotenoid spectrum, as described in Example 6.

For the spectrophotometric analysis, 0.5 ml the broth was diluted with 3 volumes of water, and centrifuged. The pellet fraction was resuspended with 1.5 ml of 96% ethanol, and centrifuged.

The pellet was transferred to a Potter tube, and treated with chloroform until all the color had been extracted into the chloroform fraction. The chloroform fraction was filtered to remove particulate material. A portion of the extract was diluted 100-fold with acetone for absorption measurement at 507 nm against an acetone blank. After 5 days of fermentation, the level of lycopene was found to be 1.36 and 1.60 g/I in duplicate measurements. The dry matter content of the broth was 42 g/kg, so the lycopene content of the biomass was 3.5% on average.

### Example 6. Analysis of the carotenoid spectrum

Frozen samples obtained in Example 5 were thawed. Glass beads (5 g) were added to 250 mg of sample, and the mixture was vigorously shaken for 30 seconds. Subsequently, 2 ml of a mixture of chloroform and pyridine (200 : 1 by volume) were added, and the samples were vigorously shaken for 90 minutes. Then 8 ml of a solution of the antioxidant BHT (butylated hydroxy-toluene) in acetone (100 mg/l) were added, and the whole sample was mixed thoroughly. Subsequently, 5 ml of this solution was centrifuged 5 minutes at 3000 rpm, and the carotenoid spectrum was determined in the supernatant fraction by HPLC, with detection of the various peaks at multiple wavelengths, using a Beckman Ultrasphere ODS column, and a mixture of 800 ml acetonitrile, 200 ml methanol, 80 ml hexane and 1 ml triethylamine as mobile phase.

The wavelengths used for detecting specific carotenoids and the Extinction Coefficients used for the correction are given in Table 9 below.

**Table 9: Detection wave-lengths (nm) and Extinction Coefficients (E.C.)**

| | β-carotene | | | lycopene | | γ-carotene | Neurosporene | phytoene |
|---|---|---|---|---|---|---|---|---|
| | Trans | 9-cis | 13-cis | trans | cis | | | |
| Wavelength | 454 | 454 | 454 | 505 | 505 | 469 | 469 | 290 |
| E.C. | 2592 | 2174 | 1655 | 3150 | 1150 | 2600 | 2600 | 800 |

The relative amounts of the carotenoids were calculated from the absorption at their specific wavelength, corrected for the differences in their extinction coefficients. The following relative amounts of the carotenoids were found as presented in Table 10.

**Table 10: Carotenoids mixture produced by the mutant**

| Carotenoid | Relative amount |
|---|---|
| trans-Lycopene | 78.3 % |
| cis-Lycopene | 4.6 % |
| trans-beta-Carotene | 10.4 % |
| 13-cis-beta-Carotene | 0.3 % |
| gamma-Carotene | 3.5 % |
| Neurosporene | 1.1 % |
| Phytoene | 1.9 % |

It is clear that lycopene is the most abundant carotenoid produced by *B. trispora*-strain *lyc26* (-). The form of lycopene produced by the biosynthetic pathway of B. trispora is the trans-stereoisomer. Isomerisation to the cis-form occurs to various extents during sample processing or isolation of the Lycopene, depending on the processing conditions. The relative amount of total lycopene produced (trans and cis) is 82.9%. The relative amount d total β-carotene produced (trans and cis) is 10.7%. We found a lycopene to β-carotene ratio of 7.7.

### Example 7. Comparison of carotenoid spectrum between lycopene and β-carotene roduction media in shake flasks

A shake flask experiment was performed according to the procedure described in Example 3, using the same strains. A parallel experiment was done, using the same inoculation and seed culture procedure, but employing a production medium suitable for β-carotene. This β-carotene production medium had the following composition as given in Table 11.

**Table 11: β-carotene production medium (in % w/v).**

| | |
|---|---|
| dextrose (Boom) | 3 |
| corn steep solids (Roquette) | 3.15 |
| KH2PO4 | 0.05 |
| thiamine | 0.0002 |
| corn oil (Cerestar) | 3.8 |
| pH adjusted to 6.0 - 6.5 with KOH | |
| sterilization at 120°C for 40 minutes | |

The main difference between the lycopene production medium of Example 3 and the β-carcitene production medium of this Example is the much higher level of sugars in the lycopene production medium.The ratio of lycopene to β-carotene produced in both media is shown in Table 12.

**Table 12: Influence of the medium on the ratio of lycopene to β-carotene**

| Medium | Ratio of lycopene to β-carotene |
|---|---|
| lycopene medium as given in Example 3 | 5.2 |
| β-carotene medium as given in Example 7 | 0.53 |

We conclude the type of medium used influences the carotenoid spectrum produced by the mutant lyc 26; the lycopene medium of Example 3 containing high level of sugar being particularly suited for obtaining high yield of lycopene.

### Example 8. Isolation of lycopene by extraction

3 Liters of fermentation broth, produced in a 10 L fermenter according to the procedure of Example 5, were filtered using a Seitz 2 liter filter. The filter cake was washed with 1800 ml of process water. Subsequently, the washed cake was extruded and dried overnight at 40 °C under vacuum.

About 100 g of biomass was recovered with a dry matter content of 78 %. The biomass was extracted with subsequent portions of 500 ml of fresh ethyl acetate at 40 °C for 10 minutes. In total, 8 extraction cycles were performed, finally resulting in 3800 g of extract.

The extract was filtered and concentrated at 40 °C until the final volume was about 500 ml. The heating was stopped and evaporation was continued by an on-going reduction of the pressure. At a temperature of 9 °C the operation was stopped. The resulting suspension was filtered using a G2 glass filter. Crystals in the shape of needles were observed.

The crystals were washed at ambient temperature with 100 ml of fresh ethyl acetate, followed by a wash with 100 ml of fresh ethanol. Finally 0.23 g of crystals were recovered after drying overnight at 40 °C. The lycopene content of the crystals was 50 - 80 %, 4 - 8% of the lycopene being the cis-isomer.

### Example 9. Direct isolation of lycopene

2 Liters of fermentation broth, produced in a 10 L fermenter according to the procedure of Example 5, were homogenized by 3 passages at 500 bars through an APV Lab60 homogenizer. To this homogenized broth, 0.3 volumes of 1-butanol were added, and the mixture was centrifuged for 5 minutes at 5000 rpm. The same centrifugation conditions were also used for all subsequent centrifugation steps. The interface fraction was collected, and washed with 100 volumes of 1-butanol. The mixture was centrifuged, and the pellet fraction was collected. A mixture was prepared consisting of pellet, 1-butanol and water at a volumetric ratio of 1:2:10. The pH was adjusted to 11.5 - 12.6 with NaOH, and the mixture was incubated for 2 hours at 80 °C. Sodium acetate was added to a final concentration of 50 g/l, and the mixture was centrifuged. Subsequently, 10 volumes of water were added to the top layer, the pH of the top layer was adjusted to 3:5 with HCl, and the mixture was incubated for 45 minutes at ambient temperature. Subsequently, 0.1 volumes of 1-butanol were added, and the whole mixture was centrifuged. The interface was collected, and 10 volumes of ethanol were added. The mixture was centrifuged, and the pellet fraction was collected. This fraction was washed with 10 volumes of ethanol, centrifuged, and dried for 145 minutes at 40 °C under vacuum.

The crystals thus obtained were found to contain 80% trans-lycopene, 1 % cis-lycopene and 3.5% β-carotene.

## Claims

1. A strain VKPM F-822 of Blakeslea trispora, wherein the strain produces at least 0.3g/l lycopene, when co-cultivated for at least three days with a suitable strain of Blakeslea trispora of the opposite mating type in a suitable medium in the absence of an exogenous specific carotenogenesis inhibitor.

## Patentansprüche

1. VKPM-F-822-Stamm von Blakeslea trispora, wobei der Stamm, wenn er mindestens drei Tage lang mit einem geeigneten Stamm von Blakeslea trispora des entgegengesetzten Kreuzungstyps in einem geeigneten Medium in Abwesenheit eines exogenen spezifischen Karotinogenesehemmers co-kultiviert wird, mindestens 0,3 g/l Lycopen produziert.

## Revendications

1. Souche VKPM F-822 de Blakeslea trispora, **caractérisée en ce que** la souche produit au moins 0,3 g/l de lycopène lorsqu'elle est co-cultivée pendant au moins trois jours avec une souche convenable de Blakeslea trispora de type sexuel opposé dans un milieu convenable en absence d'un inhibiteur de caroténogénèse spécifique exogène.
